# EUROPEAN PATENT SPECIFICATION

(11) **EP 2 240 477 B1**
(45) Date of publication and mention of the grant of the patent: **10.09.2014**
(21) Application number: 09701042.5
(22) Date of filing: 09.01.2009
(51) Int. Cl.: C07D 451/10

(54) **NOVEL PROCESS FOR THE PREPARATION OF SCOPINE ESTERS**
NEUARTIGES VERFAHREN ZUR HERSTELLUNG VON SCOPIN-ESTERN
NOUVEAU PROCÉDÉ DE PRÉPARATION D'ESTERS DE SCOPINE

(30) Priority: 10.01.2008 IN KO00772008
(43) Date of publication of application: 20.10.2010
(73) Proprietor: Generics [UK] Limited, Potters Bar Hertfordshire EN6 1AG (GB); Mylan Development Centre Private Limited, Maharashtra 410208 (IN)
(72) Inventor: GAITONDE, Abhay, Maharashtra 410208 (IN); MANOJKUMAR, Bindu, Maharashtra 410208 (IN); SHINDE, Dattatraya, Maharashtra 410208 (IN); TANK, Sinderpal, Maharashtra 410208 (IN)
(74) Representative: Elend, Almut Susanne
(86) International application number: PCT/GB2009/050014
(87) International publication number: WO 2009/087419

(56) References cited:
- EP-A- 0 418 716
- WO-A-03/057694
- WO-A-2007/012626
- WO-A-2008/089852
- WO-A-2008/104955
- US-A1- 2004 242 622
- US-A1- 2007 167 480

## Description

### Field of the invention

The present invention relates to novel processes for the preparation of scopine esters and their quaternary salts. In particular, the present invention relates to a process for the preparation of tiotropium bromide.

### Background of the invention

Tiotropium bromide (1), first disclosed in European Patent Application EP418716, is a highly effective anticholinergic agent with a specificity for muscarinic receptors and it is presently approved for the treatment of respiratory disorders, such as asthma or chronic obstructive pulmonary disease (COPD), including chronic bronchitis and emphysema.

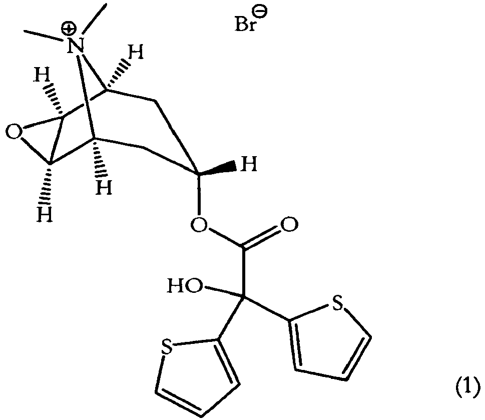

Tiotropium bromide is used in low (microgram) therapeutic doses and it is therefore particularly necessary to develop an industrial process for the commercial preparation of tiotropium bromide which ensures that the product is prepared not only in a good, economical yield but also with exceptional purity.

A process for the preparation of tiotropium bromide was first reported in EP418716. This method of synthesising tiotropium bromide describes, in a first step, the transesterification reaction of scopine (2) with methyl di(2-thienyl)glycolate (4) to form the di(2-thienyl)glycolic acid scopine ester (3), which we will refer to in this application as tiotropium base. The ester (3) is then quaternised with methyl bromide to form tiotropium bromide. However, hazardous reagents such as sodium metal are used for the transesterification step to form tiotropium base (3). In addition, the yields for the preparation of the tiotropium base (3) are low with an HPLC purity around 45-50% - the remaining impurity being di(2-thienyl)glycolic acid (5). The reported process is also inconvenient as the tiotropium base (3) needs to be isolated and purified before quaternisation to afford tiotropium bromide (1).

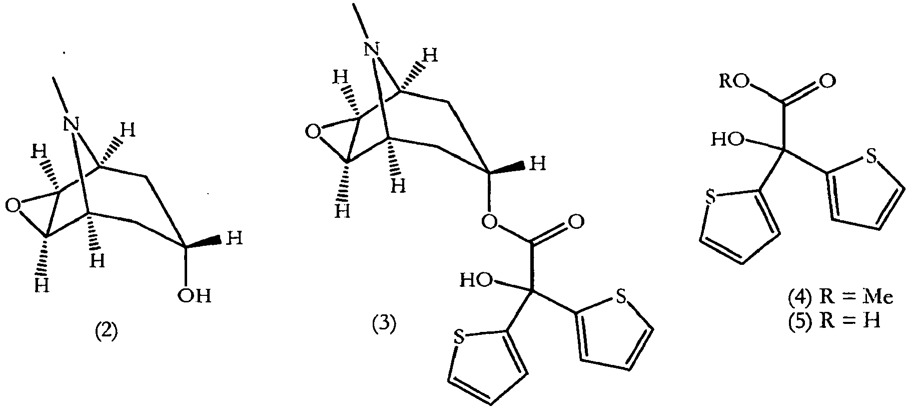

Alternative processes, reported in US patents US6486321, US6506900, US6610849 and US6747153, describe the preparation of tiotropium bromide starting from tropenol hydrochloride. However, these processes are not convenient as they are complex procedures involving a number of synthetic steps and require an epoxidation reaction later in the synthetic route to form the scopine ester moiety.

An alternative process, reported in US patent US6747154, describes the preparation of tiotropium bromide via a direct coupling reaction between di(2-thienyl)glycolic acid and the quaternised derivative scopine methyl bromide. Although this is a short, direct synthesis, the method requires the use of expensive coupling agents such as carbonyldiimidazole, carbonyldi-1,2,4-triazole, ethyldimethylaminopropylcarbodiimide or dicyclohexyl-carbodiimide. In addition, there are other disadvantages to this method as the reaction proceeds at low (sub-zero) temperature, hazardous reagents such as lithium hydride have to be employed, and yields of the purified product are modest.

The present inventors were interested in preparing highly pure tiotropium and related compounds by the most convenient and shortest route, which avoids the use of hazardous and/or environmentally unsuitable reagents.

The processes reported in the prior art, as described above, are not very efficient or convenient for commercial manufacture of pure product and an alternative method is required.

### Object of the invention

It is therefore an object of the present invention to provide an efficient, simple and non-hazardous process for the preparation of tiotropium bromide (1), tiotropium base (3) and related compounds.

### Definitions

For the purposes of the present invention, an "alkyl" group is defined as a monovalent saturated hydrocarbon, which may be straight-chained or branched, or be or include cyclic groups. An alkyl group may optionally be substituted, and may optionally include one or more heteroatoms N, O or S in its carbon skeleton. Preferably an alkyl group is straight-chained or branched. Preferably an alkyl group is not substituted. Preferably an alkyl group does not include any heteroatoms in its carbon skeleton. Examples of alkyl groups are methyl, ethyl, n-propyl, i-propyl, n-butyl, i-butyl, t-butyl, n-pentyl, cyclopentyl, cyclohexyl and cycloheptyl groups. Preferably an alkyl group is a C₁₋₁₂ alkyle group, preferably a C₁₋₆ alkyl group. Preferably a cyclic alkyle group is a C₃₋₁₂ cyclic alkyl group, preferably a C₅₋₇ cyclic alkyl group.

An "alkenyl" group is defined as a monovalent hydrocarbon, which comprises at least one carbon-carbon double bond, which may be straight-chained or branched, or be or include cyclic groups. An alkenyl group may optionally be substituted, and may optionally include one or more heteroatoms N, O or S in its carbon skeleton. Preferably an alkynyl group is straight-chained or branched. Preferably an alkenyl group is not substituted. Preferably an alkenyl group does not include any heteroatoms in its carbon skeleton. Examples of alkenyl groups are vinyl, allyl, but-1-enyl, but-2-enyl, cyclohexenyl and cycloheptenyl groups. Preferably an alkynyl group is a C₂₋₁₂ alkenyl group, preferably a C₁₋₆ alkenyl group. Preferably a cyclic alkenyl group is a C₃₋₁₂ cyclic alkenyl group, preferably a C₅₋₇ cyclic alkenyl group.

An "alkynyl" group is defined as a monovalent hydrocarbon, which comprises at least one carbon-carbon triple bond, which may be straight-chained or branched, or be or include cyclic groups. An alkynyl group may optionally be substituted, and may optionally include one or more heteroatoms N, O or S in its carbon skeleton. Preferably an alkynyl group is straight-chained or branched. Preferably an alkynyl group is not substituted. Preferably an alkynyl group does not include any heteroatoms in its carbon skeleton. Examples of alkynyl groups are ethynyl, propargyl, but-1-ynyl and but-2-ynyl groups. Preferably an alkynyl group is a C₂₋₁₂ alkynyl group, preferably a C₂₋₆ alkynyl group. Preferably a cyclic alkynyl group is a C₃₋₁₂ cyclic alkynyl group, preferably a C₅₋₇ cyclic alkynyl group.

An "aryl" group is defined as a monovalent aromatic hydrocarbon. An aryl group may optionally be substituted, and may optionally include one or more heteroatoms N, O or S in its carbon skeleton. Preferably an aryl group is not substituted. Preferably an aryl group does not include any heteroatoms in its carbon skeleton. Examples of aryl groups are phenyl, naphthyl, anthracenyl, phenanthrenyl, thienyl and furyl groups. Preferably an aryl group is a C₄₋₁₄ aryl group, preferably a C₆₋₁₀ aryl group.

For the purposes of the present invention, where a combination of groups is referred to as one moiety, for example, arylalkyl, arylalkenyl, arylalkynyl, alkylaryl, alkenylaryl or alkynylaryl, the last mentioned group contains the atom by which the moiety is attached to the rest of the molecule. A typical example of an arylalkyl group is benzyl

An "alkoxy" group is defined as a -O-alkyl, -O-alkenyl, -O-alkynyl, -O-aryl, -O-arylalkyl, -O-arylalkenyl, -O-arylalkynyl, -O-alkylaryl, -O-alkenylaryl or -O-alkynylaryl group. Preferably an "alkoxy" group is a -O-alkyl or -O-aryl group. More preferably an "alkoxy" group is a -O-alkyl group.

A "halo" group is a tluoro, chloro, bromo or iodo group.

For the purposes of this invention, an optionally substituted group may be substituted with one or more of -F, -Cl, -Br, -I, -CF₃, -CCl₃, -CBr₃, -CI₃, -OH, -SH, -NH₂, -CN, -NO₂, -COOH, -R^{a}-O-R^{b}, -R^{a}-S-R^{b}, -R^{a}-N(R^{b})₂, -R^{a}-N(R^{b})₃⁺, -R^{a}-P(R^{b})₂, -R^{a}-Si(R^{b})₃, -R^{a}-CO-R^{b}, -R^{a}-CO-OR^{b}, -R^{a}O-CO-R^{b}, -R^{a}-CO-N(R^{b})₂, -R^{a}-NR^{b}-CO-R^{b}, -RO-CO-OR^{b}, -R^{a}O-CO-N(R^{b})₂, -R^{a}-NR^{b}-CO-OR^{b}, -R^{a}-NR^{b}-CO-N(R^{b})₂, -R^{a}-CS-R^{b} or -R^{b}. In this context, -R^{a}- is independently a chemical bond, a C₁-C₁₀ alkylene, C₂-C₁₀ alkenylene or C₂-C₁₀ alkynylene group. -R^{b} is independently hydrogen, unsubstituted C₁-C₆ alkyl or unsubstituted C₆-C₁₀ aryl. Preferably an optionally substituted group may be substituted with one or more of C₁-C₄ alkyl, C₁-C₄ alkoxy, hydroxy, halo or haloalkyl, all unsubstituted. Optional substituent(s) are not taken into account when calculating the total number of carbon atoms in the parent group substituted with the optional substituent(s). Preferably a substituted group comprises 1, 2 or 3 substituents, more preferably 1 or 2 substituents, and even more preferably 1 substituent.

For the purposes of the present invention, a compound is "substantially pure", if it comprises less than 1% impurity by HPLC, preferably less than 0.5%, preferably less than 0.3%, preferably less than 0.2%, preferably less than 0.1%.

### Summary of the invention

A first aspect of the present invention provides a process for the preparation of the scopine ester I or its quaternary salt II: 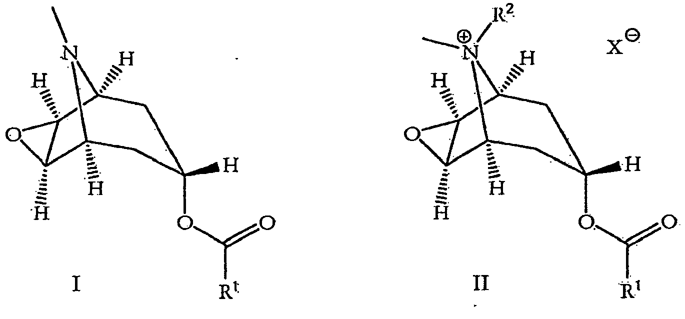 comprising transesterification of scopine, or a mineral acid or organic acid addition salt thereof, with a suitable carboxylic ester R¹CO₂R³; wherein R¹ and R² independently represent hydrogen, alkyl, alkenyl, alkynyl, optionally substituted aryl, or optionally substituted arylalkyl, arylalkenyl, arylalkynyl, alkylaryl, alkenylaryl or alkynylaryl; R³ represents alkyl, alkenyl, alkenyl, optionally substituted aryl, or optionally substituted arylalkyl, arylalkenyl, arylalhynyl, alkylaryl, alkenylaryl or alkynylaryl; and X represents a pharmaceutically acceptable anion;
wherein the transesterification reaction is performed in the presence of an organic amine base;
wherein an "alkyl" group is a monovalent saturated hydrocarbon, which may be straight-chained or branched, or be or include cyclic groups, and which may optionally be substituted, and which may optionally include one or more heteroatoms N, O or S in its carbon skeleton;
wherein an "alkenyl" group is a monovalent hydrocarbon, which comprises at least one carbon-carbon double bond, and which may be straight-chained or branched, or be or include cyclic groups, and which may optionally be substituted, and which may optionally include one or more heteroatoms N, O or S in its carbon skeleton;
wherein an "alkynyl" group is a monovalent hydrocarbon, which comprises at least one carbon-carbon triple bond, and which may be straight-chained or branched, or be or include cyclic groups, and which may optionally be substituted, and which may optionally include one or more heteroatoms N, O or S in its carbon skeleton; and
wherein an "aryl" group is a monovalent aromatic hydrocarbon, which may optionally be substituted, and which may optionally include one or more heteroatoms N, O or S. in its carbon skeleton.

In a preferred process R¹ is represented by formula III, wherein R⁴, R⁵ and R⁶ independently represent hydrogen, hydroxy, halo, alkoxy, alkyl, hydroxyalkyl, alkenyl, alkynyl, optionally substituted aryl, or optionally substituted arylalkyl, arylalkenyl, arylalkynyl, alkylaryl, alkenylaryl or alkynylaryl.

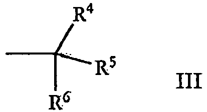

Preferably R⁴ and/or R⁵ represent aryl, wherein the aryl group can be selected from phenyl, naphthyl, thienyl and furyl, which may optionally be mono- or disubstituted by one or two groups selected from C₁-C₄ alkyl, C₁-C₄ alkoxy, hydroxy, halo or haloalkyl. Most preferably the aryl group is 2-thienyl.

Preferably R⁶ represents hydroxy, C₁-C₄ alkyl, C₁-C₄ alkoxy, hydroxyalkyl, halo or haloalkyl.

Most preferably, R⁴ is 2-thienyl, R⁵ is 2-thienyl and R⁶ is hydroxy.

Preferably R² represents hydrogen or C₁-C₄ alkyl, more preferably methyl.

Preferably R³ represents C₁-C₄ alkyl, and most preferably R³ represents methyl.

Preferably, X represents halo, methanesulfonate, toluenesulfonate or trifluoromethanesulfonate. Most preferably X represents a bromo.

Most preferably when forming the quaternary salt II, R² is methyl and X is bromo.

Preferably the scopine is used in the form of a salt, preferably an acid addition salt, and most preferably in the form of its hydrochloride salt

The transesterification reaction is performed in the presence of an organic amine base. The organic amine base is preferably a trialkylamine such as triethylamine or diisopropylethylamine, or a heterocyclic amine such as 1,8-diazabicyclo[5.4.0]undec-7-ene (DBU), 1,5-diazabicyclo[4.3.0]non-5-ene (DBN), 1,4-dia;sabicyclo[2.2.2]octane (Dabco), pyridine or 4-(dimethylamino)pyridine (DMAP). Most preferably, the organic amine base is DBU. Preferably 1-5 equivalents of the organic amine base are used relative to the scopine or the salt thereof, preferably 1-3 equivalents of the organic amine base are used.

Additionally a further base may be used for the transesterification reaction. Preferably the further base is an inorganic base, preferably a hydride such as NaH, KH or CaH₂. Preferably the further base is NaH. Preferably 1-2 equivalents of the further base are used relative to the scopine or the salt thereof.

If two bases are used, they may be used in any order, i.e. the further base may be added to the reaction mixture before, after and/or simultaneous with the first base. Without wishing to be bound by theory, the base(s) are believed to deprotonate the scopine hydroxyl group or a protonated reaction intermediate. Moreover, if the scopine is used in the form of a salt, the base(s), in particular the inorganic base, are believed to liberate scopine free base in situ.

The reaction temperature used in the transesterification step is preferably in the range of 30 to 90°C, more preferably in the range of 40 to 70°C, and more preferably in the range of 50 to 70°C. Most preferably the reaction is carried out at about 60°C.

Preferably, the process according to the first aspect of the invention is carried out such that formation of the quaternary salt II is obtained without purification and/or isolation of ester I.

Preferably, the transesterification reaction is carried out in polar aprotic solvent, such as a solvent selected from dimethylformamide, dimethylsulfoxide, acetonitrile or N-methylpyrrolidine. Preferably, the solvent is dimethylformamide.

Preferably, the scopine ester I or its quaternary salt II are obtained in an HPLC purity of greater than 95%, preferably greater than 98%, preferably greater than 99%, preferably greater than 99.5%, preferably greater than 99.7%, preferably greater than 99.8%, more preferably greater than 99.9%.

Preferably, the scopine ester I or its quaternary salt II are obtained in a yield of greater than 50%, preferably greater than 55%, more preferably greater than 60%.

### Detailed description of the invention

Surprisingly, it has been found that tiotropium base (3) and tiotropium bromide (1) can be obtained in substantially pure form when synthesised by the efficient and more advantageous process of the present invention. A preferred embodiment of the present invention is outlined in Scheme 1.

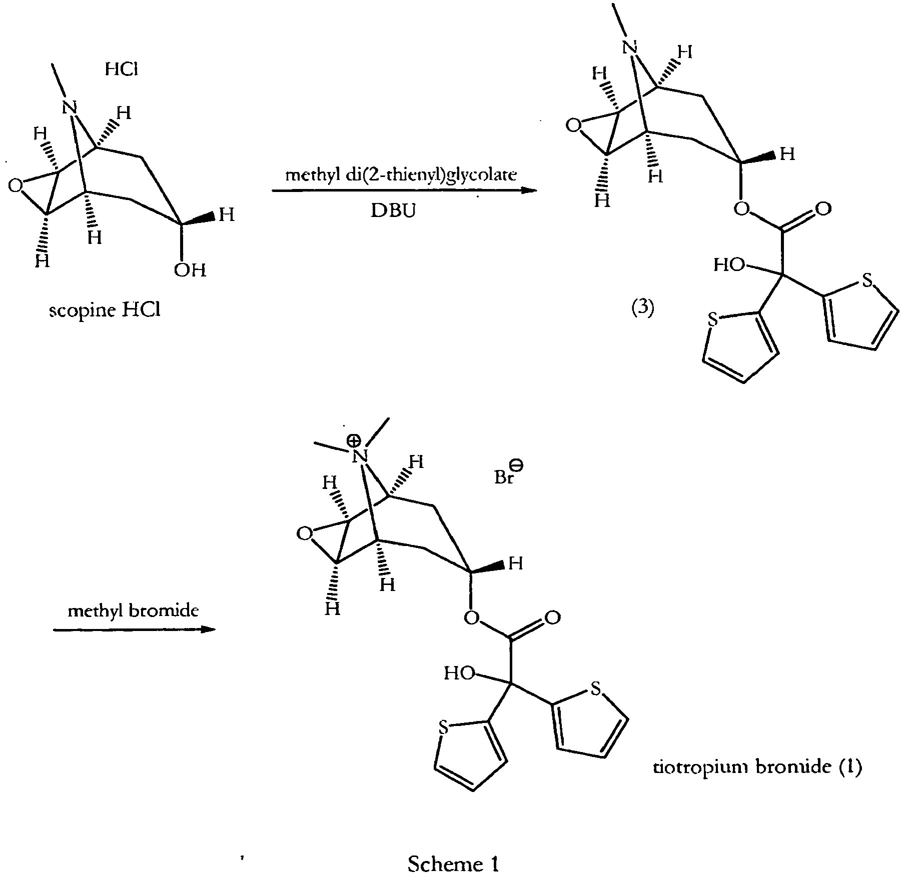

The process outlined in Scheme 1 is a novel process for the preparation of tiotropium base (3) and is very advantageous as the use of DBU (1,8-diazabicyclo[5.4.0]undec-7-ene) as base gives a dramatic improvement in the purity from 45-50% (obtained when using prior art processes) to >99.6% by HPLC. The overall yield is also improved over the prior art. The solvent used is preferably DMF and the reaction is preferably carried out at about 60°C.

Preferably, the solvent used in the transesterification step is DMF, but alternatively the solvent used can be dimethylsulfoxide, acetonitrile or N-methylpyrrolidine.

The reaction temperature used in the transesterification step is preferably in the range of 30 to 90°C, more preferably in the range of 40 to 70°C, and more preferably in the range of 50 to 70°C. Most preferably the reaction is carried out at about 60°C.

The tiotropium base (3) formed by the present invention is so pure that it can surprisingly be quaternised, for example with methyl bromide, without isolation and purification to afford a highly pure quaternary salt product. This is a huge benefit in a commercial operation as it saves very significantly on time and cost if a purification and/or isolation step can be avoided.

The organic amine bases used in the present invention are preferably trialkylamines such as diisopropylethylamine or triethylamine, or a heterocyclic amine such as 1,8-diazabicyclo[5.4.0]undec-7-ene (DBU), 1,5-dimibicyclo[4.3.0]non-5-ene (DBN), 1,4-diazabicyclo[2.2.2]octane (Dabco), pyridine or 4-(dimethylamino)pyridine (DMAP). The organic base used is most preferably DBU.

Preferably the scopine is used in the form of its hydrochloride salt, but alternatively it can be used in the form of the free base or another mineral acid addition salt (e.g. HBr or HI) or an organic acid addition salt (e.g. acetate, benzoate, propionate, maleate, fumarate, oxalate, besylate, mesylate, tosylate, citrate of salicylate).

Other preferred embodiments of the first aspect of the invention are the preparation of scopolamine [represented by ester I, wherein R¹ is represented by formula III and R⁴ is hydrogen, R⁵ is phenyl and R⁶ is hydroxymethyl (CH₂OH)]; scopolamine hydrogen bromide [represented by salt II, wherein R¹ is represented by formula III and R⁴ is hydrogen, R⁵ is phenyl, R⁶ is hydroxyethyl R² is hydrogen and X is bromo]; oxitropium bromide [represented by salt II, wherein R¹ is represented by formula III and R⁴ is hydrogen, R⁵ is phenyl, R⁶ is hydroxymethyl, R² is ethyl and X is bromo]; and cimetropium bromide [represented by salt II, wherein R¹ is represented by formula III and R⁴ is hydrogen, R⁵ is phenyl, R⁶ is hydroxyethyl, R² is -CH₂-cyclopropyl and X is bromo].

A pharmaceutical composition may comprise tiotropium bromide prepared according to the first aspect of the invention. Preferably, the pharmaceutical composition is suitable for use in a dry powder inhaler (DPI), an aqueous nebulizer, or a pressurized metered dosage inhaler (pMDI).

The DPI compositions preferably contain, in addition to the active substance, one or more of the following physiologically acceptable excipients: monosaccharides (e.g. glucose or arabinose), disaccharides (e.g. lactose, sucrose, maltose), oligo- and polysaccharides (e.g. dextrane), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly in the form of their hydrates. Lactose is a particularly preferred excipient, while lactose monohydrate is most particularly preferred.

Preferably the pMDI uses HFA 134a, HFA 227 or mixtures thereof as propellant gas.

The pharmaceutical composition can also be a solution, suspension or a solid oral dosage form if so desired. Preferred oral dosage forms include tablets, capsules and the like which, optionally, may be coated if desired. Tablets can be prepared by conventional techniques, including direct compression, wet granulation and dry granulation. Capsules are generally formed from a gelatine material and can include a conventionally prepared granulate of excipients.

The pharmaceutical composition typically comprises one or more conventional pharmaceutically acceptable excipient(s) selected from the group comprising a filler, a binder, a disintegrant, a lubricant and optionally further comprises at least one excipient selected from colouring agents, adsorbents, surfactants, film formers and plasticizers.

If the solid pharmaceutical formulation is in the form of coated tablets, the coating may be prepared from at least one film-former such as hydroxypropyl methyl cellulose, hydroxypropyl cellulose or methacrylate polymers which optionally may contain at least one plasticizer such as polyethylene glycols, dibutyl sebacate, triethyl citrate, and other pharmaceutical auxiliary substances conventional for film coatings, such as pigments, fillers and others.

The pharmaceutical compositions preferably contain about 0.001 to 20% tiotropium bromide in admixture with a physiologically acceptable excipient. Preferred compositions contain 0.01 to 10% of tiotropium bromide, more preferred are compositions which contain 0.01 to 2% of tiotropium bromide, and most preferred are compositions which contain 0.04 to 0.8% of tiotropium bromide.

The following examples are provided to illustrate the present invention and should not be construed as limiting thereof.

### Examples

### Tiotropium base (3)

Scopine HCl was taken in DMF (5vol), cooled to 5°C, and NaH (1.7eq) was added slowly maintaining the temperature at 5°C. The reaction was stirred for 1 hour at 10°C and DBU (1eq) and methyl di(2-thienyl)glycolate (1eq) were added. The reaction was heated to 60°C for 1 hour and a second portion of DBU (2eq) was added. The reaction was heated for a further 4 hours at 60°C and monitored by TLC. After completion of the reaction, the mixture was cooled to 5°C and a solution of conc. HCl (2.5vol) in cold water (10vol) at 10°C (pH 2) was added. The mixture was washed with toluene and basified with aqueous sodium carbonate (7.5eq) to pH 10 and extracted with DCM (3 x 10vol). The combined DCM layer was washed with water (3 x 10vol) and DCM was distilled under vacuum (150 mbar) at 30°C. The product was obtained as light brown solid. Molar Yield = 60%; HPLC purity = 98%.

The crude base (3) was recrystallized from acetonitrile (5vol). Yield of crystallization = 86%; HPLC purity > 99.8%.

### Tiotropium bromide (1)

The purified tiotropium base (3) was dissolved in DCM (10vol) and acetonitrile (3vol) and purged with methyl bromide gas at a pressure of 10 kg/cm² for 20 minutes. The solution was kept at 25-30°C for 30 hours. The precipitated solid was filtered and washed with DCM (20vol). Drying of the solid at 25-30°C under vacuum gave the product as a white solid. Molar Yield = 97.76%; HPLC purity = 99.83%.

¹H-NMR (300 MHz, CD₃OD): 7.45 (2H dd), 7.23 (2H dd), 7.00 (2H dd), 5.27 (1H t), 4.60 (1H br s OH), 3.35 (8H m), 3.10 (2H s), 2.85 (2H dt), 2.10 (2H d). MS: 392.3 (M+1)

### Alternative process for the preparation of tiotropium bromide (1) without isolation of tiotropium base (3)

Scopine HCl was taken in DMF (5vol), cooled to 5°C, and NaH (1.7eq) was added slowly maintaining the temperature at 5°C. The reaction mixture was stirred for 1 hour at 10°C and DBU (1eq) and methyl di(2-thienyl)glycolate (1eq) were added. The reaction was heated to 60°C for 1 hour and a second lot of DBU (2eq) was added. The reaction mixture was heated for a further 4 hours at 60°C and monitored by TLC. After completion of the reaction, the mixture was cooled to 5°C and a solution of conc. HCl (2.5vol) in cold water (10vol) at 10°C (pH 2) was added. The mixture was washed with toluene (1vol) and the aqueous layer was basified with saturated sodium carbonate (7.5eq) solution to pH 10 and extracted with DCM (3 x 10vol). The combined DCM layer was washed with water (3 x 10vol) and dried over anhydrous sodium sulfate.

To the DCM solution, acetonitrile was added and the mixture was purged with methyl bromide gas at a pressure of 3 kg/cm² for 30 minutes. The solution was kept at 25-30°C for 30 hours. The precipitated solid was filtered and washed with DCM (20vol). Drying of the solid at 25-30°C under vacuum gave the product as a white solid. Molar yield = 41%; HPLC purity = 98.66%.

## Claims

1. A process for the preparation of scopine ester I or its quaternary salt II: 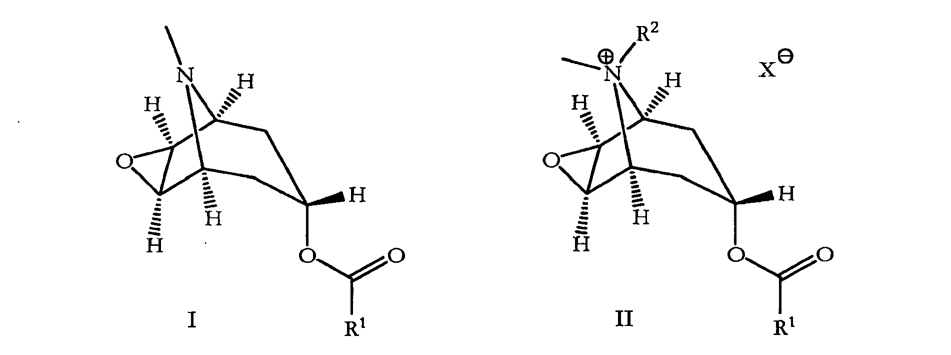 comprising transesterification of scopine, or a mineral acid or organic acid addition salt thereof, with a suitable carboxylic ester represented by formula R¹CO₂R³; wherein R¹ and R² independently represent hydrogen, alkyl, alkenyl, alkenyl, optionally substituted aryl, or optionally substituted arylalkyl, arylalkenyl, arylalkynyl, alkylaryl, alkenylaryl or alkynylaryl; R³ represents alkyl, alkenyl, alkynyl, optionally substituted aryl, or optionally substituted arylalkyl, arylalkenyl, arylalkynyl, alkylaryl, alkenylaryl or alkenylaryl; and X represents a pharmaceutically acceptable anion; wherein the transesterification reaction is performed in the presence of an organic amine base;
wherein an "alkyl" group is a monovalent saturated hydrocarbon, which may be straight-chained or branched, or be or include cyclic groups, and which may optionally be substituted, and which may optionally include one or more heteroatoms N, O or S in its carbon skeleton;
wherein an "alkenyl" group is a monovalent hydrocarbon, which comprises at least one carbon-carbon double bond, and which may be straight-chained or branched, or be or include cyclic groups, and which may optionally be substituted, and which may optionally include one or more heteroatoms N, O or S in its carbon skeleton;
wherein an "alkynyl" group is a monovalent hydrocarbon, which comprises at least one carbon-carbon triple bond, and which may be straight-chained or branched, or be or include cyclic groups, and which may optionally be substituted, and which may optionally include one or more heteroatoms N, O or S in its carbon skeleton; and
wherein an "aryl" group is a monovalent aromatic hydrocarbon, which may optionally be substituted, and which may optionally include one or more heteroatoms N, O or S in its carbon skeleton.

2. A process according to claim 1, wherein:
(i) R¹ is represented by formula III: 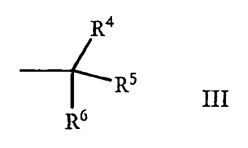 wherein R⁴, R⁵ and R⁶ independently represent hydrogen, hydroxy, halo, alkoxy, alkyl, hydroxyalkyl, alkenyl, alkynyl, optionally substituted aryl, or optionally substituted arylalkyl, arylalkenyl, arylalkynyl, alkylaryl, alkenylaryl or alkynylaryl; and/or
(ii) R⁷ represents hydrogen or C₁-C₄ alkyl; and/or
(iii) R³ represents C₁-C₄ alkyl; and/or
(iv) R³ represents methyl; and/or
(v) X represents a halo, a methanesulfonate, a toluenesulfonate or a trifluoromethanesulfonate group; and/or
(vi) X represents a bromo group; and/or
(vii) R² is methyl and X is bromo.

3. A process according to claim 2, wherein:
(i) R⁴ and/or R⁵ represent aryl; and/or
(ii) R⁴ and/or R⁵ represent aryl, and wherein the aryl group is selected from phenyl, naphthyl, thienyl and furyl, which may optionally be mono- or disubstituted by one or two groups selected from C₁-C₄ alkyl, C₁-C₄ alkoxy, hydroxy, halo or haloalkyl; and/or
(iii) R⁴ and/or R⁵ represent aryl, and wherein the aryl group is 2-thienyl; and/or
(iv) R⁶ represents hydroxy, C₁-C₄ alkyl, C₁-C₄ alkoxy, hydroxyalkyl, halo or haloalkyl; and/or
(v) R⁴ is 2-thienyl, R⁵ is 2-thienyl and R⁶ is hydroxyl.

4. A process according to any one of the preceding claims, wherein the organic amine base is:
(i) a trialkylamine or a heterocyclic amine; and/or
(ii) selected from triethylamine, diisopropylethylamine, 1,8-diazabicyclo[5.4.0]undec-7-ene (DBU), 1,5-diazabicyclo[4.3.0]non-5-ene (DBN), 1,4-diazabicyclo[2.2.2]octane (Dabco), pyridine or 4-(dimethylamino)pyridine (DMAP); and/or
(iii) DBU.

5. A process according to any one of the preceding claims, wherein a further base is used.

6. A process according to claim 5, wherein the further base is:
(i) an inorganic base; and/or
(ii) a hydride; and/or
(iii) NaH, KH or CaH₂; and/or
(iv) NaH.

7. A process according to claim 5 or 6, wherein the scopine is:
(i) used in the form of a salt; and/or
(ii) used in the form of a salt, and wherein the further base is used to liberate scopine free base in situ.

8. A process according to any one of the preceding claims, wherein:
(i) the scopine is used in the form of its hydrochloride salt; and/or
(ii) formation of the quaternary salt II is carried out without purification and/or isolation of ester I; and/or
(iii) the transesterification reaction is carried out in dimethylformamide; and/or
(iv) the scopine ester I or its quaternary salt II are obtained in an HPLC purity of greater than 95%; and/or
(v) the scopine ester I or its quaternary salt II are obtained in a yield of greater than 50%.

## Patentansprüche

1. Verfahren zur Herstellung von Scopinester I oder dessen quartären Salz II: 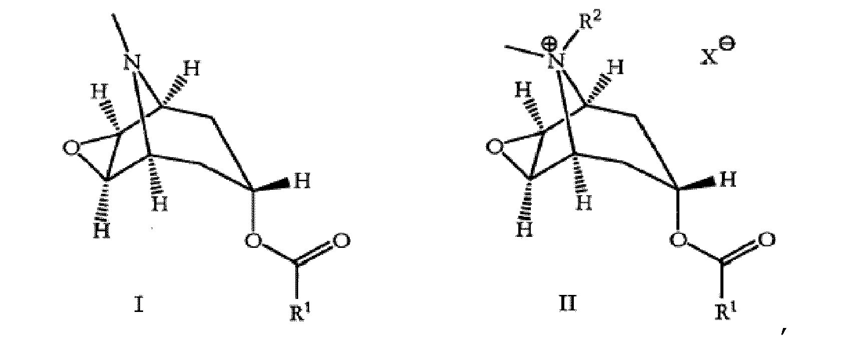 wobei das Verfahren die Umesterung von Scopin oder eines Mineralsäure- oder organischen Säureadditionssalzes davon mit einem geeigneten Carbonsäureester umfasst, der durch die Formel R¹CO₂R³ dargestellt wird; wobei R¹ und R² unabhängig für Wasserstoff, Alkyl, Alkenyl, Alkinyl, gegebenenfalls substituiertes Aryl oder gegebenenfalls substituiertes Arylalkyl, Arylalkenyl, Arylalkinyl, Alkylaryl, Alkenylaryl oder Alkinylaryl stehen; R³ für Alkyl, Alkenyl, Alkinyl, gegebenenfalls substituiertes Aryl oder gegebenenfalls substituiertes Arylalkyl, Arylalkenyl, Arylalkinyl, Alkylaryl, Alkenylaryl oder Alkinylaryl steht und X für ein pharmazeutisch unbedenkliches Anion steht; wobei die Umesterungsreaktion in Gegenwart einer organischen Aminbase durchgeführt wird;
wobei eine "Alkyl"-Gruppe ein einwertiger gesättigter Kohlenwasserstoff ist, der geradkettig oder verzweigt sein kann oder cyclische Gruppen sein oder enthalten kann und der gegebenenfalls substituiert sein kann und der gegebenenfalls ein oder mehrere Heteroatome N, 0 oder S in seinem Kohlenstoffgerüst enthalten kann; wobei eine "Alkenyl"-Gruppe ein einwertiger Kohlenwasserstoff ist, der mindestens eine Kohlenstoff-Kohlenstoff-Doppelbindung umfasst und der geradkettig oder verzweigt sein kann oder cyclische Gruppen sein oder enthalten kann und der gegebenenfalls substituiert sein kann und der gegebenenfalls ein oder mehrere Heteroatome N, 0 oder S in seinem Kohlenstoffgerüst enthalten kann;
wobei eine "Alkinyl"-Gruppe ein einwertiger Kohlenwasserstoff ist, der mindestens eine Kohlenstoff-Kohlenstoff-Dreifachbindung umfasst und der geradkettig oder verzweigt sein kann oder cyclische Gruppen sein oder enthalten kann und der gegebenenfalls substituiert sein kann und der gegebenenfalls ein oder mehrere Heteroatome N, 0 oder S in seinem Kohlenstoffgerüst enthalten kann; und
wobei eine "Aryl"-Gruppe ein einwertiger aromatischer Kohlenwasserstoff ist, der gegebenenfalls substituiert sein kann und der gegebenenfalls ein oder mehrere Heteroatome N, 0 oder S in seinem Kohlenstoffgerüst enthalten kann.

2. Verfahren nach Anspruch 1, wobei:
(i) R¹ durch die Formel III dargestellt wird: 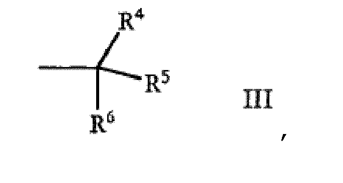 wobei R⁴, R⁵ und R⁶ unabhängig für Wasserstoff, Hydroxy, Halo, Alkoxy, Alkyl, Hydroxyalkyl, Alkenyl, Alkinyl, gegebenenfalls substituiertes Aryl oder gegebenenfalls substituiertes Arylalkyl, Arylalkenyl, Arylalkinyl, Alkylaryl, Alkenylaryl oder Alkinylaryl stehen und/oder
(ii) R² für Wasserstoff oder C₁-C₄-Alkyl steht und/oder
(iii) R³ für C₁-C₄-Alkyl steht und/oder
(iv) R³ für Methyl steht und/oder
(v) X für eine Halo-, eine Methansulfonat-, eine Toluolsulfonat- oder eine Trifluormethansulfonatgruppe steht und/oder
(vi) X für eine Bromgruppe steht und/oder
(vii) R² Methyl ist und X Brom ist.

3. Verfahren nach Anspruch 2, wobei:
(i) R⁴ und/oder R⁵ für Aryl stehen und/oder
(ii) R⁴ und/oder R⁵ für Aryl stehen und wobei die Arylgruppe aus Phenyl, Naphthyl, Thienyl und Furyl ausgewählt ist, das gegebenenfalls durch eine oder zwei Gruppen, die aus C₁-C₄-Alkyl, C₁-C₄-Alkoxy, Hydroxy, Halo oder Haloalkyl ausgewählt ist bzw. sind, mono- oder disubstituiert sein kann; und/oder
(iii) R⁴ und/oder R⁵ für Aryl stehen und wobei die Arylgruppe 2-Thienyl ist und/oder
(iv) R⁶ für Hydroxy, C₁-C₄-Alkyl, C₁-C₄-Alkoxy, Hydroxyalkyl, Halo oder Haloalkyl steht und/oder
(v) R⁴ 2-Thienyl ist, R⁵ 2-Thienyl ist und R⁶ Hydroxyl ist.

4. Verfahren nach einem der vorhergehenden Ansprüche, wobei die organische Aminbase:
(i) ein Trialkylamin oder ein heterocyclisches Amin ist und/oder
(ii) aus Triethylamin, Diisopropylethylamin, 1,8-Diazabicyclo[5.4.0]undec-7-en (DBU), 1,5-Diazabicyclo[4.3.0]non-5-en (DBN), 1,4-Diazabicyclo[2.2.2]octan (Dabco), Pyridin oder 4-(Dimethylamino)pyridin (DMAP) ausgewählt ist und/oder
(iii) DBU ist.

5. Verfahren nach einem der vorhergehenden Ansprüche, wobei eine weitere Base verwendet wird.

6. Verfahren nach Anspruch 5, wobei die weitere Base:
(i) eine anorganische Base und/oder
(ii) ein Hydrid und/oder
(iii) NaH, KH oder CaH₂ und/oder
(iv) NaH ist.

7. Verfahren nach Anspruch 5 oder 6, wobei das Scopin:
(i) in der Form eines Salzes verwendet wird und/oder
(ii) in der Form eines Salzes verwendet wird und wobei die weitere Base dazu verwendet wird, die freie Base von Scopin *in situ* freizusetzen.

8. Verfahren nach einem der vorhergehenden Ansprüche, wobei:
(i) das Scopin in der Form seines Hydrochloridsalzes verwendet wird und/oder
(ii) die Bildung des quartären Salzes II ohne Aufreinigung und/oder Isolierung des Esters I durchgeführt wird und/oder
(iii) die Umesterungsreaktion in Dimethylformamid durchgeführt wird und/oder
(iv) der Scopinester I oder dessen quartäres Salz II in einer HPLC-Reinheit von mehr als 95 % erhalten wird und/oder
(v) der Scopinester I oder dessen quartäres Salz II in einer Ausbeute von mehr als 50 % erhalten wird.

## Revendications

1. Procédé pour la préparation d'ester de scopine I ou de son sel quaternaire II : 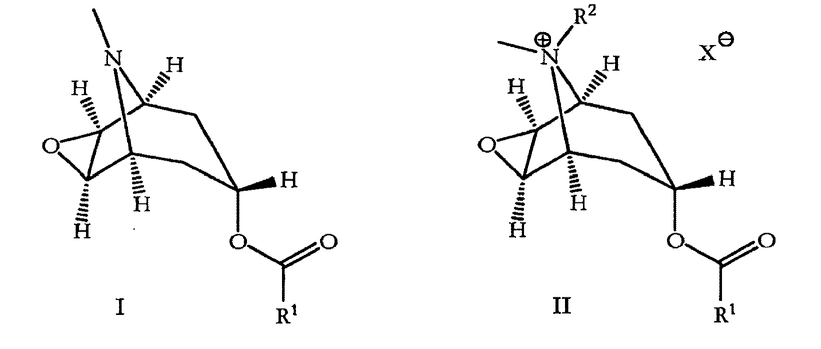 comprenant la transestérification de scopine, ou d'un sel d'addition d'acide minéral ou d'acide organique de celle-ci, avec un ester carboxylique approprié représenté par la formule R¹CO₂R³ ; où R¹ et R² représentent indépendamment hydrogène, alkyle, alcényle, alcynyle, aryle facultativement substitué ou arylalkyle facultativement substitué, arylalcényle, arylalcynyle, alkylaryle, alcénylaryle ou alcynylaryle ; R³ représente alkyle, alcényle, alcynyle, aryle facultativement substitué ou arylalkyle facultativement substitué, arylalcényle, arylalcynyle, alkylaryle, alcénylaryle ou alcynylaryle ; et X représente un anion pharmaceutiquement acceptable ; dans lequel la réaction d'transestérification est effectuée en présence d'une base amine organique ;
où un groupe « alkyle » est un hydrocarbure saturé monovalent, qui peut être à chaîne droite ou ramifié, ou qui peut être ou comprendre des groupes cycliques, et qui peut facultativement être substitué, et qui peut facultativement comprendre un ou plusieurs hétéroatomes N, 0 ou S dans son squelette carboné ;
où un groupe « alcényle » est un hydrocarbure monovalent, qui comprend au moins une double liaison carbone-carbone, et qui peut être à chaîne droite ou ramifié, ou qui peut être ou comprendre des groupes cycliques, et qui peut facultativement être substitué, et qui peut facultativement comprendre un ou plusieurs hétéroatomes N, O ou S dans son squelette carboné ;
où un groupe « alcynyle » est un hydrocarbure monovalent, qui comprend au moins une triple liaison carbone-carbone, et qui peut être à chaîne droite ou ramifié, ou qui peut être ou comprendre des groupes cycliques, et qui peut facultativement être substitué, et qui peut facultativement comprendre un ou plusieurs hétéroatomes N, 0 ou S dans son squelette carboné ; et où un groupe « aryle » est un hydrocarbure aromatique monovalent, qui peut facultativement être substitué, et qui peut facultativement comprendre un ou plusieurs hétéroatomes N, 0 ou S dans son squelette carboné.

2. Procédé selon la revendication 1, dans lequel :
(i) R¹ est représenté par la formule III : 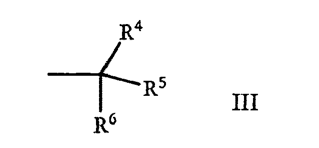 où R⁴, R⁵ et R⁶ représentent indépendamment hydrogène, hydroxy, halogéno, alcoxy, alkyle, hydroxyalkyle, alcényle, alcynyle, aryle facultativement substitué ou arylalkyle facultativement substitué, arylalcényle, arylalcynyle, alkylaryle, alcénylaryle ou alcynylaryle ; et/ou
(ii) R² représente hydrogène ou alkyle en C₁-C₄ ; et/ou
(iii) R³ représente alkyle en C₁-C₄ ; et/ou
(iv) R³ représente méthyle ; et/ou
(v) X représente un groupe halogéno, méthanesulfonate, toluènesulfonate ou trifluorométhanesulfonate ; et/ou
(vi) X représente un groupe bromo ; et/ou
(vii) R² est méthyle et X est bromo.

3. Procédé selon la revendication 2, dans lequel :
(i) R⁴ et/ou R⁵ représentent aryle ; et/ou
(ii) R⁴ et/ou R⁵ représentent aryle et dans lequel le groupe aryle est choisi parmi phényle, naphtyle, thiényle et furyle, qui peut facultativement être monosubstitué ou disubstitué par un ou deux groupes choisis parmi alkyle en C₁-C₄, alcoxy en C₁-C₄, hydroxy, halogéno ou halogénoalkyle ; et/ou
(iii) R⁴ et/ou R⁵ représentent aryle et dans lequel le groupe aryle est 2-thiényle ; et/ou
(iv) R⁶ représente hydroxy, alkyle en C₁-C₄, alcoxy en C₁-C₄, hydroxyalkyle, halogéno ou halogénoalkyle ; et/ou
(v) R⁴ est 2-thiényle, R⁵ est 2-thiényle et R⁶ est hydroxyle.

4. Procédé selon l'une quelconque des revendications précédentes, dans lequel la base amine organique est :
(i) une trialkylamine ou une amine hétérocyclique ; et/ou
(ii) choisie parmi la triéthylamine, la diisopropyléthylamine, le 1,8-diazabicyclo[5.4.0]undéc-7-ène (DBU), le 1,5-diazabicyclo[4.3.0]non-5-ène (DBN), le 1,4-diazabicyclo[2.2.2]octane (Dabco), la pyridine ou la 4-(diméthylamino)pyridine (DMAP) ; et/ou
(iii) le DBU.

5. Procédé selon l'une quelconque des revendications précédentes, dans lequel une autre base est utilisée.

6. Procédé selon la revendication 5, dans lequel l'autre base est :
(i) une base inorganique ; et/ou
(ii) un hydrure ; et/ou
(iii) NaH, KH ou CaH₂ ; et/ou
(iv) NaH.

7. Procédé selon la revendication 5 ou 6, dans lequel la scopine est :
(i) utilisée sous la forme d'un sel ; et/ou
(ii) utilisée sous la forme d'un sel et dans lequel l'autre base est utilisée pour libérer la base libre de scopine *in situ.*

8. Procédé selon l'une quelconque des revendications précédentes, dans lequel :
(i) la scopine est utilisée sous la forme de son sel chlorhydrate ; et/ou
(ii) la formation du sel quaternaire II est effectuée sans purification et/ou isolement de l'ester I ; et/ou
(iii) la réaction de transestérification est effectuée dans du diméthylformamide ; et/ou
(iv) l'ester de scopine I ou son sel quaternaire II sont obtenus à une pureté par HPLC supérieure à 95 % ; et/ou
(v) l'ester de scopine I ou son sel quaternaire II sont obtenus avec un rendement supérieur à 50 %.
